# EUROPEAN PATENT SPECIFICATION

(11) **EP 1 425 305 B1**
(45) Date of publication and mention of the grant of the patent: **29.12.2010**
(21) Application number: 02755576.2
(22) Date of filing: 10.09.2002
(51) Int. Cl.: A61K 39/21, A61K 39/39, C07K 14/65, C07K 14/155

(54) **IGF-1 AS FELINE VACCINE ADJUVANT, IN PARTICULAR AGAINST FELINE RETROVIRUSES**
IGF-1 ALS IMPFSTOFFHILFSMITTEL FÜR KATZEN, INSBESONDERE GEGEN FELINE RETROVIREN
IGF-1 UTILISE COMME ADJUVANT DE VACCIN FELIN, NOTAMMENT CONTRE LES RETROVIRUS FELINS

(30) Priority: 11.09.2001 FR 0111736
(43) Date of publication of application: 09.06.2004
(73) Proprietor: MERIAL, 69007 Lyon (FR)
(72) Inventor: ANDREONI, Christine, Michèle, Pierrette, F-38280 Villette D'Anthon (FR)
(74) Representative: Harding, Charles Thomas
(86) International application number: PCT/IB2002/003638
(87) International publication number: WO 2003/022886

(56) References cited:
- EP-A- 0 229 750
- WO-A-01/36483
- WO-A-86/00619
- WO-A-95/16703
- WO-A-97/33997
- WO-A-98/03660
- WO-A-98/24922
- US-A- 5 202 119
- US-A- 5 473 054
- WOO J C ET AL: "Investigation of recombinant human insulin-like growth factor type I in thymus regeneration in the acute stage of experimental FIV infection in juvenile cats." 10 October 1999 (1999-10-10), AIDS RESEARCH AND HUMAN RETROVIRUSES 10 OCT 1999 LNKD- PUBMED:10515153, VOL. 15, NR. 15, PAGE(S) 1377 - 1388 ISSN: 0889-2229
- VOGEL AND POWELL: COMPENDIUM OF VACCINE ADJUVANTS AND EXCIPIENTS, 1995, pages 141-228,
- ALVING ET AL: "novel adjuvant strategies for experimental malaria and AIDS vaccines" ANN NY ACAD SCI, no. 690, 1993, pages 265-275,
- WOO J C; DEAN G A; LAVOY A; CLARK R; MOORE P F: "nvestigation of recombinant human insulin-like growth factor type I in thymus regeneration in the acute stage of experimental FIV infection in juvenile cats." AIDS RESEARCH AND HUMAN RETROVIRUSSES, vol. 15, no. 15, 10 October 1999 (1999-10-10), pages 1377-1388,

## Description

The present invention relates to an immunogenic composition or vaccine against a feline disease, comprising a feline immunogen or a recombinant vector expressing in vivo a feline immunogen, wherein said feline immunogen is an FIV immunogen; and a polypeptide having an IGF-1 activity, or an in vivo expression vector comprising a polunucleotide encoding a polypeptide having an IGF-1 activity, and a veterinary acceptable vehicle or excipient. The present invention further relates to the use of said composition for the prevention and/or treatment of infection by FIV.

The present description relates to the feline growth factor IGF-1, to a polynucleotide encoding this IGF-1 and to expression vectors containing it. The description also relates to the use of IGF-1 as vaccine adjuvant for the feline species, in particular for vaccines against feline retroviruses.

IGF-1 is an endocrine growth mediator. It is the main effector of the growth hormone. IGF-1 also plays a part in the complex system of regulation of immunity and of inflammation on the sides of cytokines, of hormones (in particular cortisol, the growth hormone).

US-A-5,202,119 describes the therapeutic use of human IGF-1 as an immunity stimulant in man and, more particularly, in immunodeficient individuals. A particular application is the combination of human IGF-1 with an immunogen to stimulate the production of antibodies to this immunogen. This patent aims to extend the application to mammals in general and to the avian species, but without describing animal IGF-1 sequences or providing the results of experiments on animals.

Alving et al (1993; Annals New York Academy of Sciences, 690: 265-275) is a review article focusing on adjuvant development and vaccine delivery systems that provide new tools for amplifying the effectiveness of ongoing malaria and AIDS vaccine development programs.

The Applicant has found that IGF-1 stimulates more particularly the cellular response in general. This response profile has turned out to be particularly well adapted in the case of feline retroviruses. It also stimulates mucosal immunity.

Until the invention, moreover, the gene encoding feline IGF-1 was not available.

The applicant has succeeded in isolating and sequencing the IGF-1 gene originating from cats' peripheral blood mononuclear cells (BPMC). This gene was isolated after a polymerase chain reaction (PCR) using the oligonucleotides described in the examples.

The feline IGF-1 gene has a size of 462 nucleotides (SEQ ID NO:1) and codes for a protein containing 153 amino acids (SEQ ID NO: 2). At the N-terminal of the protein as defined in SEQ ID NO: 2 there is a signal peptide sequence of 48 amino acids (from 1 to 48 in the sequence SEQ ID NO: 2) serving for the secretion of the protein, this signal peptide being cleaved secondarily. The mature protein (SEQ ID NO: 3) of 105 amino acids corresponds to the amino acids 49 to 153 of SEQ ID NO: 2.

The present invention provides in one aspect an immunogenic composition or vaccine against a feline disease, comprising a feline immunogen or a recombinant vector expressing *in vivo* a feline immunogen, wherein said feline immunogen is an FIV immunogen;
and a polypeptide having an IGF-1 activity, the polypeptide being selected from:
(i) a polypeptide comprising the amino acid sequence as depicted in SEQ ID NO: 2 or SEQ ID NO: 3,
(ii) a polypeptide encoded by nucleotide sequence as depicted in SEQ ID NO: 1,
(iii) a polypeptide comprising amino acids 49-118 on SEQ ID NO: 2,
(iv) a polypeptide having identity equal to or higher than 98.5 % with a polypeptide according to i, ii or iii;
or an *in vivo* expression vector comprising a polynucleotide encoding a polypeptide having an IGF-1 activity, the polynucleotide being selected from:
(i) a polynucleotide comprising a nucleotide sequence as depicted in SEQ ID NO: 1 or its complementary strand,
(ii) a polynucleotide encoding a polypeptide having the amino acid sequence as depicted in SEQ ID NO: 2 or 3,
(iii) a polynucleotide comprising a sequence which differs from the sequence SEQ ID NO: 1 due to the replacement of T by U,
(iv) a polynucleotide encoding amino acids 49-118 of SEQ ID NO: 2,
(v) a polynucleotide encoding a polypeptide having identity equal to or higher than 98.5 % with a polypeptide according to ii or iv;
and a veterinary acceptable vehicle or excipient.

In another aspect, the present invention provides the use of a composition according to the present invention in the preparation of a medicament for the prevention and/or treatment of infection by FIV.

In a further aspect, the present invention provides a composition according to the present invention for use in the prevention and/or treatment of infection by FIV.

There is mention herein the DNA sequence encoding feline IGF-1 and its complementary strand and to the corresponding RNA sequence, and to the equivalent sequences obtained by degeneracy of the genetic code.

The first object of the description is therefore an isolated polynucleotide (DNA, in particular cDNA, or RNA, by definition) comprising nucleotide sequence SEQ ID NO: 1 or the same sequence with substitution of the T by U to form the RNA sequence. A further subject is the isolated polynucleotide having this sequence. The present description also relates to an isolated polynucleotide encoding amino acid sequence SEQ ID NO: 2 or 3.

By definition, the notion of IGF-1 according to the description covers the IGF-1 of feline origin and the proteins having identity equal to or higher than 98.5% and preferably 99% with SEQ ID NO: 2. The polynucleotides coding for these proteins of feline origin or the homologues thus defined form part of the description.

Thus the description mentions a polynucleotide encoding a polypeptide or protein having the function of feline IGF-1, selected from the group comprising:
(a) a polynucleotide comprising or consisting essentially of nucleotide sequence as depicted in SEQ ID NO: 1 or its complementary strand
(b) a polynucleotide encoding a polypeptide having the amino acid sequence as depicted in SEQ ID NO: 2 or 3
(c) a polynucleotide the sequence of which differs from the sequence of (a) due to the replacement of T by U.

By way of equivalence, this includes a polynucleotide encoding a polypeptide or protein having the function of feline IGF-1, such as:
(d) a polynucleotide the sequence of which differs from the sequence of (a) or (b) due to the degeneracy of the genetic code
(e) polynucleotide encoding a polypeptide having identity equal to or higher than 98.5% and preferably than 99% with SEQ ID NO: 2.

The percentage of identity is determined using the Basic Local Alignment Search Tool (BLAST) (Altschul et al., J. Mol. Biol. 215:403, 1990), this program is available from several sources, including the National Center for Biotechnology Information (NCBI, Bethesda, MD) and on the Internet. The comparison between the sequences is made over the full length alignment with the amino acid sequence given in this present disclosure, employing the NCBI Blast. 2 sequences function using the blastp default BLOSUM62 matrix set to default parameters (gap existence cost of 11, and a per residue gap cost of 1).

The polynucleotide according to the description may be obtained by chemical synthesis or by expression using appropriate expression vectors.

The present description also mentions the feline IGF-1 proteins or polypeptides isolated or produced in mature forms or their precursors. The description therefore relates, in particular, to:
(f) a polypeptide comprising or consisting essentially of SEQ ID NO: 2 or SEQ ID NO: 3,
(g) a polypeptide encoded by SEQ ID NO: 1 or by any one of polynucleotides (a) to (c) above.

By way of equivalence, this includes a protein or polypeptide having the function of IGF-1, wherein the polypeptide has identity equal to or higher than 98.5% and preferably than 99% with SEQ ID NO: 2.

The GF-1 protein or polypeptide may be expressed and isolated in mature form in a system of cells allowing the secretion thereof and the cleavage of the signal peptide at 5'. It may also be expressed and isolated in precursor form.

The present description also relates to the expression vectors containing a polynucleotide as defined above as an insert, and the elements allowing expression of the polynucleotide.

The nucleotide sequence may be inserted into conventional *in vitro* expression vectors, in particular of viral origin such as *Baculovirus* or of plasmid origin. These vectors are then used to infect or transfect appropriate systems of cells such as insect cells, cells of prokaryotic origin (for example *Escherichia coli*) or eukaryotic origin, in particular yeasts, in particular *Saccharomyces cerevisiae,* mammalian cells, in particular hamster cells (for example CHO cells) and cat cells (for example CRFK cells). It is preferable to use the *Baculovirus* (U5-A-4,745,051; Wialard J. et al., J. Virol., 1990, 64(1), 37-50; Verne A., Virology, 1988, 167, 56-71) e.g. Autographa californica Nuclear Polyhedrosis Virus AcNPV as vector to express the IGF-1 in insect cells; in particular cells of Spodoptera frugiperda Sf9 (ATCC CRL 1711 deposit). A person skilled in the art is familiar with and able to use the methods of producing proteins adapted to the selected system and thus obtain and isolate the IGF-1 protein. The description therefore also covers these *in vitro* expression vectors comprising a polynucleotide according to the description and the elements allowing expression thereof in the host cells, the feline IGF-1 protein thus produced and its use as vaccine adjuvant and non-specific immunity stimulant.

Preferably, the polynucleotide according to the description is introduced into *in vivo* expression vectors under conditions allowing the expression of a functional polypeptide or feline IGF-1 protein in the host. These expression vectors may be plasmids, viral vectors such as poxviruses, for example attenuated mutants of the vaccinia virus, avipox (in particular canarypox, fowlpox), swinepox, raccoonpox, adenoviruses (in particular CAV-2) and herpes viruses such as the feline herpes virus FHV. Of the viral vectors, canarypox and FHV are preferred. These vectors comprise the elements allowing the expression of the polynucleotide in the host.

With regard to the poxviruses, a person skilled in the art can refer to WO-A-90/12882 and, more particularly, with regard to the vaccinia virus to US-A-4,769,330; US-A-4,722,848; US-A-4,603,112; US-A-5,110,587; US-A-5,494,807, US-A-5,762,938; with regard to fowlpox to US-A-5,174,993; US-A-5,505,941; US-A-5,766,599; and with regard to canarypox to US-A-5;756,103.

As attenuated mutant of vaccinia virus, one may mention the MVA (Ankara strain) (Stickl H. and Hochstein-Mintzel V., Munch, Med. Wschr., 1971, 113, 1149-1153; Sutter G. et al., Proc. Natl. Acad. Sci. USA., 1992, 89, 10847-10851; commercial strain ATCC VR-1508; MVA is obtained after 570 passages of the Ankara vaccinia strain on chicken embryo fibroblasts), or the NYVAC (its construction is described in US-A-5,494,807, in particular in examples 1 to 6; this patent also describes insertion of heterologous genes within insertion sites in this recombinant, and the use of appropriate promoters; see also WO-A-96/40241).

According to one of the preferred embodiments of the invention, the poxvirus expression vector is a canarypox virus, optionally attenuated, e.g. an ALVAC or a canarypox virus (for example of the Rentschler strain) which has been attenuated, in particular by more than 200 passages on chick embryo fibroblast (CEF) cells. An ALVAC strain canarypox virus was registered, on 14 November 1996, with the American Type Culture Collection (ATCC) under the accession number VR-2547. A canarypox is commercially available at the ATCC under reference VR-111. Attenuated canarypox viruses are described in US-A-5,756,103 and WO-A-01/05934.

Other attenuated poxviruses may be used, in particular attenuated fowlpoxes (e.g. TROVAC). Regarding the TROVAC poxvirus, those skilled in the art may refer to patent WO-A-96/40241. A number of fowlpox vaccinal strains are available, e.g. the vaccine DIFTOSEC CT^{®} sold by Merial and the vaccine NOBILIS^{®} sold by Intervet.

If the expression vector is an attenuated mutant of the vaccinia virus, the insertion sites of the polynucleotide(s) to be expressed are, in particular, the thymidine kinase (TK) gene, the hemagglutinin (HA) gene and the region of the type A inclusion bodies (ATI). Insertion of genes in the MVA virus is also described in several publications, e.g. in M. W. Carroll et al., Vaccine 1997, 15(4), 387-394; K.J. Stittelaar et al., J. Virol. 2000, 74(9), 4236-4243; G. Sutter et al., Vaccine 1994,12(11), 1032-1040, to which the one skilled in the art may refer. The complete genome of MVA is described in G. Antoine, Virology 1998, 244, 365-396, which allows one to find other insertion sites and other promoters.

In the case of canarypox, the insertion sites are, in particular, located in or formed by the open reading frames (ORFs) C3, C5 and C6. In the case of fowlpox, the insertion sites are, in particular, located in or formed by the ORFs F7 and F8.

Preferably, if the expression vector is a poxvirus, the polynucleotide to be expressed is inserted under the control of a poxvirus specific promoter, in particular the vaccinia 7.5 kDa promoter (Cochran et al., J. Virology, 1985, 54, 30-35), the vaccinia I3L promoter (Riviere et al., J. Virology, 1992, 66, 3424-3434), the vaccinia HA promoter (Shida, Virology, 1986, 150, 451-4.57), the cowpox ATI promoter (Funahashi et al., J. Gen. Virol., 1988, 69, 35-47), or else the vaccinia H6promoter (Taylor J. et al., Vaccine, 1988, 6, 504-508; Guo P. et al., J. Virol., 1989, 63, 4189-4198; Perkus M. et al, J. Virol., 1989, 63, 3829-3836).

If the expression vector is a FHV, the sites of insertion of the polynucleotide(s) to be expressed are, in particular, the open reading frames (ORFs) ORF2 and ORF5 (US-A-6,074,649).

Preferably, if the expression vector is a herpes virus, the polynucleotide to be expressed is inserted under the dependency of signals regulating the transcription and in particular of promoters, preferably brought by the insertion, for example the early promoter of the cytomegalovirus or CMV-IE (Cytomegalovirus Immediate Early), in particular of human origin (hCMV-IE), from rats, guinea pigs or preferably of murine origin (mCMV IE), the early and late promoters of the virus SV40 (Simian Virus 40), the late promoters of glycoproteins, in particular of gB, gC and gD, of alpha herpes viruses, in particular of feline herpes viruses (FHV).

By definition, a plasmid comprises at least one transcription unit comprising a polynucleotide of interest, for example the one encoding IGF-1 and the elements required for the *in vivo* expression thereof. The circular, super-coiled or otherwise, plasmid form is preferred. The linearised form also falls within the scope of this description.

Each plasmid comprises a promoter capable of ensuring, in host cells, the expression of the polynucleotide inserted under its control. It is, in general, a strong eukaryotic promoter. The cytomegalovirus immediate early (CMV-IE) promoter, of human or murine origin, or optionally of any other origin such as rat or guinea pig, is the preferred strong eukaryotic promoter. The CMV-IE promoter may comprise the actual promoter component, which may or may not be associated with the enhancer component. Reference may be made to EP-A-260 148, EP-A-323 597, US-A-5 168 062, US-A-5 385 839, US-A-4 968 615 and WO-A-87/03905. Human (Boshart M. et al., Cell., 1985, 41, 521-530) or murine CMV-IE is preferred.

More generally, the promoter is either of viral origin or of cellular origin. As a strong viral promoter other than CMV-IE, mention may be made of the early promoter or the late promoter of the virus SV40, or the LTR promoter of the Rous sarcoma virus. As a strong cellular promoters, mention may be made of the cytoskeleton gene promoter, such as, the desmin promoter (Kwissa M. et al., Vaccine, 2000, 18(22), 2337-2344) or else the actin promoter (Miyazaki J. et al., Gene, 1989, 79(2), 269-277).

If several genes are present in the same plasmid, they may be presented in the same transcription unit or in several different units.

By equivalence, the sub-fragments of these promoters, maintaining adequate promoter activity, are included in the present description: e.g. the truncated CMV-IE promoters according to WO-A-98/00166. The notion of promoter according to the description therefore includes the derivatives and sub-fragments maintaining adequate promoter activity, preferably substantially similar to that of the proper promoter from which they are derived. With regard to CMV-IE, this notion includes the proper promoter part and/or the enhancer part and the derivatives and sub-fragments.

The expression vectors can also incorporate other transcription-regulating elements such as stabilising sequences of the intron type, e.g. intron II of the rabbit beta-globin gene (van Ooyen et al. Science, 1979, 206: 337-344) and/or a signal sequence, e.g. of the human tissue plasminogen activator gene (tPA; Montgomery et al., Cell. Mol. Biol. 1997, 43; 285-292), and/or a polyadenylation signal (polyA), e.g. of the gene of the bovine growth hormone (bGH) (US-A-5,122,458) or of the rabbit beta-globin gene, or that of the SV40 virus. For instance, the signal sequence of the IGF-1 may be replaced by a signal sequence of another origin.

According to the description, feline IGF-1 may be used as vaccine adjuvant, in particular to increase the cellular and/or mucosal immune response directed against one or more feline immunogens, in particular feline retrovirus immunogens such as immunogens from the feline immunodeficiency virus (FIV) and feline leukaemia virus (FeLV). IGF-1 from other species may also be used for this purpose, but feline IGF-1 is preferred.

The description therefore relates to the compositions intended to induce an immune response against a feline pathogen in a member of the feline species, in particular a cat. The immunogenic or vaccine compositions according to the description comprise an IGF-1 protein or polypeptide, preferably feline IGF-1 according to the description or a recombinant vector expressing this protein or polypeptides *in vivo,* at least one feline immunogen or a recombinant vector expressing such an immunogen *in vivo,* in particular at least one FIV and/or FeLV immunogen or a recombinant vector expressing this immunogen *in vivo,* and an excipient or vehicle which is acceptable for veterinary use. The notion of immunogenic composition includes any preparation capable of inducing an immune response directed against the pathogen under review, once administered to the feline, in particular to the cat, this response being increased by the presence of the IGF-1 protein. It is preferably a vaccine composition capable of inducing effective protection or a certain degree of protection against this pathogen, this degree of protection being increased by the presence *in vivo* of the IGF-1 protein or polypeptide, in particular feline IGF-1.

The immunogenic and vaccine compositions targeted in the invention include all known types, namely inactivated compositions, attenuated live compositions, sub-units and recombinant vectors. Thus unless indicated otherwise herein, the word immunogen encompasses an inactivated microorganism, a live attenuated microorganism, one or several microorganism subunits, or combinations thereof. The term immunogen expressed by a vector denotes in particular a peptide, polypeptide or protein capable of inducing an immune response against a pathogenic agent in the host; it may be an entire protein or glycoprotein or an immunologically active fragment thereof.

As seen hereinbefore, the IGF-1 protein or polypeptide may be added as it is to the immunogen to form, in the presence of an excipient or vehicle which is acceptable for veterinary use, a ready-to-administer composition. The IGF-1 protein may also be combined with a slow release system intended to release the protein gradually. In the immunogenic composition or vaccine, the IGF-1 protein or polypeptide can be:
i. a polypeptides as defined under (f) or (g)
ii. a polypeptide comprising or consisting essentially of amino acids 49-118 on SEQ ID NO: 2
iii. a polypeptide having identity equal to or higher than 70 %, in particular equal to or higher than 75, 80, 85, 90 or 95 %, more particularly 98.5% and preferably 99% with a polypeptide according to i or ii.

The word polypeptide includes by way of equivalence the fragments thereof which keep substantially the adjuvant power of the IGF-1, e.g. of the mature feline IGF-1 (which in the application is exemplified by amino acid sequence 49-153 or its fragment 49-118).

According to a particular feature of the description, the IGF-1 protein may be expressed *in vivo* using an *in vivo* expression vector or recombinant vector as described hereinbefore. In this case, it is also preferable to have the feline immunogen expressed by means of a recombinant vector of the same type as or of a different type from the one expressing the IGF-1 protein. A same *in vivo* expression vector, comprising and expressing at least one immunogen and the IGF-1 protein may also be used. The *in vivo* expression vector can comprise
1 a polynucleotide as defined under (a) to (c) and its equivalents
2 a polynucleotide encoding a polypeptide having identity equal to or higher than 70 %, in particular equal to or higher than 75, 80, 85, 90 or 95 %, more particularly than 98.5% and preferably than 99% with SEQ ID NO: 2
3 a polynucleotide encoding a polypeptide comprising or consisting essentially of amino acids 49-118 on SEQ ID NO: 2

Therefore, the present description preferably relates to immunogenic compositions or vaccines comprising:
- an *in vivo* expression vector containing a polynucleotide encoding a preferably feline IGF-1 under conditions allowing expression of a functional IGF-1 protein in the feline, in particular in the cat,
- at least one *in vivo* expression vector containing at least one polynucleotide encoding a FIV and/or FeLV immunogen, wherein this vector may also be the one expressing IGF-1, and
- a vehicle or excipient which is acceptable for veterinary use.

For the subunit vaccines and the recombinant vaccines, the immunogens are preferably, with regard to the FeLV virus, the envelope (env) glycoprotein or the gag/pol protein or the combinations of these FeLV immunogens and, with regard to FIV, the env, gag/pro, rev or tat proteins or combinations thereof, preferably env and gag, optionally combined with rev and/or tat. With regard to the recombinant vaccines, a nucleotide sequence encoding the immunogen under review is inserted into the expression vector under dependency of the signals required for its *in vivo* expression.

The advantages of using IGF-1 for vaccinations are, in particular, the increase in the cellular and mucosal responses and the reduction in the dose of immunogen or recombinant vector used. Furthermore, in certain animals which do not respond after administration of conventional vaccine, the use of IGF-1 in combination with the conventional vaccine stimulates the immune response and its increase to a protective level.

An advantageous feature of the invention is the use of a vector for *in vivo* expression of IGF-1 which increases the period of action of IGF-1. It is preferable to use an *in vivo* expression vector for IGF-1, whatever the form of the immunogen.

According to a particular feature, the description covers compositions comprising a plasmid encoding and expressing preferably feline IGF-1 or polypeptide according to the description and at least one further plasmid encoding and expressing at least one feline immunogen.

Examples of plasmid constructions containing feline immunogens useful in the description are given in WO-A-98/03660. As embodiments, the present description provides for the use of plasmids as disclosed in WO-A-98/03660, in particular those described in examples 7-9 (expressing FeLV immunogens env, gag/pol) and 17-18 (expressing FIV immunogens env, gag/pro) of WO-A-98/03660. And for instance, the description provides for the combination of a plasmid expressing a preferably feline IGF-1 or polypeptide and of one or several of the plasmids as disclosed in WO-A-98/03660. The description also covers compositions comprising a plasmid simultaneously encoding and expressing preferably feline IGF-1 or polypeptide according to the description and at least one FeLV or FIV immunogen. And thus the present description may use plasmids as disclosed in these examples of WO-A-98/03660, wherein a polynucleotide according to the description has been further inserted.

The description also applies to immunization against feline pathogens other than FIV and FeLV. As other pathogens, mention may be made in particular to feline panleukopenia virus (FPV), feline infectious peritonitis virus (FIPV), feline herpes Virus (FHV), feline calicivirus (FCV), rabies virus and *Chlamydia.* As shown hereinbefore, the vaccines may be attenuated vaccines, inactivated vaccines, subunit vaccines or recombinant vaccines. In the case of subunit vaccines and recombinant vaccines, the feline immunogens are preferably selected from the capsid protein VP2 for FPV, the glycoproteins S, M, N and combinations thereof for FIPV, the glycoproteins gB, gC, gD and combinations thereof for FHV, the capsid protein C for FCV and the glycoprotein G for the rabies virus.

For example, one may use the plasmids described in WO-A-98/03660 (FPV VP2 gene in example 10, FIPV S, M, N in examples 11-13, FHV gB, gD genes in examples 14-15, FCV C gene in example 16, rabies G gene in example 19) and WO-A-00177043 (FHV gB, gC, gD genes in example 5). Thus the description comprehends compositions combining such plasmid(s) and a plasmid encoding preferably feline IGF-1 protein or polypeptide. The description also comprehends compositions comprising a plasmid simultaneously encoding and expressing preferably feline IGF-1 or polypeptide according to the description and at least one feline immunogen. And thus the present description may use plasmids as disclosed in these examples of WO-A-98/03660 and WO-A-00177043, wherein a polynucleotide according to the description has been further inserted. And the above description of plasmids and viral vectors, e.g: poxviruses, will also apply to these other feline pathogens.

As other embodiments of the description the plasmids exemplified in WO-A-98/03660 and WO-A-00/77043 may also be combined with an IGF-1 or polypeptide according to the description under protein form.

This description also covers compositions in which the immunogenic or vaccine preparation comprises immunogens from two or more of these pathogens and, more particularly, immunogen(s) from one or more of these pathogens and immunogen(s) from FIV and/or FeLV.

The compositions according to the invention may also comprise one or more further immunity adjuvants selected, in particular, from those conventionally used in feline vaccination for the vaccine(s) under consideration. Conventional compositions (inactivated vaccines, attenuated live vaccines, subunit vaccines) may thus comprise, as conventional adjuvant, compounds of the carbomer type, aluminium hydroxide, or may be formulated in the form of an oil-in-water or water-in-oil-in-water emulsion. In the case of the compositions based on viral expression vector, compounds of the carbomer type and oil-in-water or water-in-oil-in-water emulsions may be cited.

The plasmid compositions may advantageously be formulated with a cationic lipid containing a quaternary ammonium salt. These lipids are preferably those corresponding to the following formula: 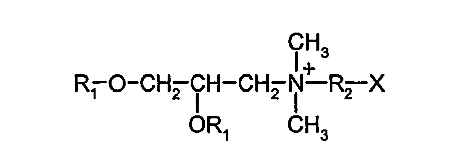 in which R₁ is a saturated or unsaturated linear aliphatic radical containing 12 to 18 carbon atoms, R₂ is a further aliphatic radical containing 2 or 3 carbon atoms and X is a hydroxyl or amine group.

Preferred cationic lipids include DMRIE (N-(2-hydroxyethyl)-N,N-dimethyl-2,3-bis(tetradecyloxy)-1-propane ammonium; WO-A-96/34109), preferably combined with a neutral lipid, preferably DOPE (dioleoyl-phosphatidyl-ethanolamine; Behr J.P., 1994, Bioconjugate Chemistry, 5, 382-389), to form DMRIE-DOPE.

Preferably, a mixture of plasmid with this adjuvant is formed extemporaneously and it is preferable to give the mixture formed in this way time to complex, for example for a period of 10 to 60 minutes, in particular of about 30 minutes, prior to administration thereof.

If DOPE is present, the molar ratio DMRIE:DOPE will preferably be 95:5 to 5:95, more particularly 1:1.

The ratio by weight of plasmid:adjuvant DMRIE or DMRIE-DOPE can range, in particular, from 50:1 to 1:10, in particular from 10:1 to 1:5, and preferably from 1:1 to 1:2.

The present description also relates to methods of immunisation and of vaccination of felines, in particular cats, in particular against the feline retroviruses FIV and FeLV.

These methods involve the administration of an effective amount of an immunogenic composition or of a vaccine according to the description. This administration may be made, in particular, parenterally, e.g. by subcutaneous, intradermal or intramuscular administration, or by oral and/or nasal routes.

These methods may be intended, in particular, to increase the cellular and/or mucosal immune response to the associated immunogen(s), in particular to FIV and/or FeLV.

The present description also relates to non-specific immunity-stimulating compositions, in other words compositions based on an IGF-1 protein or polypeptide according to the description which are be used as general immunity stimulant. The description also concerns a method for stimulating immunity comprising the administration of the composition. These compositions are administered in the presence or in the absence of declared pathology, generally independently of a vaccine, in order to reinforce immunity, in particular of a feline, preferably of a cat. These compositions preferably comprise viral or plasmid recombinant vectors which express *in vivo* an IGF-1 protein or polypeptide according to the description and an excipient or vehicle which is acceptable for veterinary use, these compositions allowing long-term production of IGF-1 in the organism. The characteristics of the vectors, polypeptides and polypeptides are as defined above. These compositions can also comprise one or more adjuvants as described hereinbefore.

The various compositions and vaccines may be injected using a needleless liquid jet injector.

The immunogenic compositions and vaccines according to the description comprise an effective amount of plasmid or viral vector, a person skilled in the art being able of determining these amounts. The Applicant recommends:
- in the case of immunogenic compositions or vaccine based on plasmid, a dose may contain from about 1 µg to about 2,000 µg, in particular from about 50 µg to about 1,000 µg. The volume of dose may be between 0.1 and 2 ml, preferably between 0.2 and 1 ml.
- in the case of the immunogenic compositions or the vaccines based on a viral vector, e.g. a poxvirus, a dose may be between about 10³ pfu and about 10³ pfu. If the vector is the canarypox virus, the dose is more particularly between about 10⁵ pfu and about 10⁹ pfu, preferably between about 10⁶ pfu and about 10⁸ pfu. If the vector is the feline herpes virus, the dose is more particularly between about 10² CCID₅₀ and about 10⁷ CCID₅₀. The volumes of dose of the immunogenic compositions and of the feline vaccines based on viral vectors are generally between 0.1 and 2.0 ml, preferably between 0.2 and 1.0 ml.

If the IGF-1 protein or polypeptide is brought in protein form, the dose may be from 20 to 2,000 µg and preferably from 100 to 500 µg.

The present invention is additionally described by the following illustrative, non-limiting examples.

### Examples

All the plasmid constructions were produced using standard molecular biology techniques (digestion by restriction enzymes, synthesis of a single strand complementary DNA, polymerase chain reaction, elongation of an oligonucleotide by a polymerase DNA, etc.) described by Sambrook J. et al. (Molecular Cloning: A Laboratory Manual, 2nd Edition, Cold Spring Harbor Laboratory, Cold Spring Harbor, New York, 1989). All restriction fragments used for the present invention as well as the various polymerase chain reaction (PCR) fragments were isolated and purified using the Geneclean® kit (BIO101 Inc. La Jolla, CA).

### Example 1: Preparation of total RNA from cat lymphocytes

Some cat's blood was collected on a heparinised tube by taking blood from the jugular vein. In the absence of stimulation, the mononuclear cells were collected by centrifugation on a Ficoll gradient. The total RNA of these cells contained in 100 ml of suspension was extracted using the High pure RNA isolation kit (Roche Molecular Biochemicals, Cat # 1 828 665), following the supplier's instructions for the extraction stages. The RNA sediment obtained at the end of extraction was resuspended with 1 to 2 ml of sterile distilled water without RNase.

### Example 2: Isolation of the gene encoding feline IGF-1

The complementary DNA (cDNA) of feline IGF-1 is synthesised with the Gene Amp RNA PCR Kit (Cat # N 808 0017, Perkin-Elmer, Norwalk, CT 06859, USA), using the conditions provided by the supplier.

A reverse transcription reaction followed by a polymerase chain reaction (RT-PCR) was carried out with 50 µl of the feline RNA suspension (example 1), the polymerase Pfu (Stratagene, Cat # 600154) and with the following oligonucleotides:
EL490 (39 mer) (SEQ ID NO: 4)
   5' ACGCCGTCGACATGGGAAAAATCAGCAGTCTTCCAACCC 3'
and FC124 (42 mer) (SEQ ID NO: 5)
   5' CGCGGATCCCTACATTCTGTAGTTCTTGTTTCCCGCACTCCC 3'

This pair of oligonucleotides allows the incorporation of a Sall restriction site, a BamHI restriction site, an ATG initiation codon at 5' of the insert and a stop codon at 3' of the insert.

The synthesis of the first cDNA strand takes place by elongation of the hexamer of the kit after hybridization of the latter with the RNA matrix.

The synthesis conditions of the first cDNA strand are a temperature of 42°C for 20 min, then 99°C for 5 min and finally 4°C for 5 min. The conditions of the PCR reaction in the presence of the pair of oligonucleotides EL490 and FC124 are a temperature of 95°C for 2 min then 40 cycles (95°C for 30 sec, then 55°C for 45 sec, and 72°C for 1 min) and finally 72°C for 10 min in the presence of the Taq DNA polymerase (Gibco BRL, Cleveland, OH, USA, Cat # 18038-26) to produce a fragment of 481 base pairs (bp).

This fragment is then inserted in the plasmid pCR2 (plasmid present in the Topo TA cloning kit, InVitrogen, Cat # K4550-40). The plasmid thus obtained has a size of 4432 bp and is designated pBP476.

All these operations are repeated twice more to obtain three "populations" of plasmids pBP476. The inserts of these plasmids are then sequenced. The consensus sequence (SEQ ID NO: 1), established on the basis of these three "populations" of plasmids pBP476 has a size of 462 bp and encoded a protein of 153 amino acids (SEQ ID NO: 2). This protein is cats' IGF-1 factor (= feline IGF-1).

### Example 3: Construction of the plasmid pBP477

The plasmid pBP476 (example 2) was digested by BamHI and SalI and the BamHI-SalI fragment of 468 bp thus obtained was ligated with the plasmid pVR1012 (Fig. 1 and example 7 of WO-A-98/03199; Hartikka J. et al, Human Gene Therapy, 1996, 7, 1205-1217), previously digested with BamHI and SalI to give the plasmid pBP477 (5334 bp). Under the control of the early promoter of the human cytomegalovirus or hCMV-IE (human Cytomegalovirus Immediate Early), this plasmid contains an insert encoding feline IGF-1.

### Example 4: In vitro biological activity of the product of the feline IGF-1 gene

CHO-K1 cells (ovarian hamster cells, available from the American Type Culture Collection under access No. CCL-61) are cultured in minimum essential medium or MEM (Gibco-BRL) in 60 mm diameter Petri dishes and are transfected with 5 µg of plasmid pBP477 (example 3), previously complexed with 10 µl of LipofectAmine PLUS® (Cat # 10964-013, Gibco-BRL, Cleveland, OH, USA). The conditions of formation of the complexes DNA/LipofectAmine® and of transfection of the cells are those recommended by the supplier (Gibco-BRL). 48 hours after transfection, the supernatants of the cultures are collected and frozen.

Some CRFK cells are cultured in plates comprising 96 wells at a rate of 100,000 cells per well in MEM medium containing an addition of 0.1% of BSA and 1% of foetal calf serum. 24 hours later, the culture medium is removed and then diluted or undiluted culture supernatant of the CHO-K1 cells transfected with the plasmid pBP477 is added to the cultures. Each dilution of the supernatant (1/1, 1/5, 1/10 and 1/100) is tested in triplicate in MEM medium. The negative control consists of a CHO-K1 culture supernatant without serum and without transfection with pBP477. After 24 hours of culture, the CRFK cells are incubated at 37°C in the presence of tritiated thymidine for the last 8 hours of culture (final tritiated thymidine concentration: 2.5 µCi/ml). The culture plates are stored at -20°C. The radioactivity is measured by reading using a MicroBeta trilux counter (Wallac).

The supernatants of CHO-K1 cells transfected with the plasmid pBP477 gave the following results:

| **Supernatant dilution** | **Number of wells** | **Average radioactivity** | **Standard deviation** | **Proliferation index** |
|---|---|---|---|---|
| Negative control | 3 | 2123 | 327 | 0% |
| 1/100 | 3 | 2872 | 657 | 135% |
| 1/10 | 3 | 8444 | 646 | 397% |
| 1/5 | 3 | 8990 | 912 | 423% |
| 1/1 | 3 | 9533 | 1091 | 449% |

The results of each test are expressed by their proliferation index corresponding to the ratio of the average radioactivity values for the sample and the negative control expressed as a percentage.

These results show that the product of the feline IGF-1 gene expressed by the plasmid pBP477 has biological activity on *in vitro* cells.

### Example 5: Culture of the FIV virus

Feline immunodeficiency viruses of the Villefranche IFFA 1/88 strain (Steffan A.M. et al., J. Gen. Virol., 1994, 75, 3647-3653) or Petaluma strain are cultivated on auxiliary feline T lymphocyte cells (for example Q201; Willet B. et al., J. Gen. Virol., 1997, 78, 611-618), for the amplification thereof.

The Q201 cells are cultured in Falcon 25 cm² with Eagle-MEM medium supplemented by 2 mM of glutamine, 10% of calf serum, 100 Ul/ml of penicillin, 100 µg/ml of streptomycin and 100 Ul/ml of recombinant human 2-interleukin containing about 100,000 cells per ml. The cells are cultivated at +37°C.

After 3 days, the cell layer is confluent. The culture medium is then replaced and the FIV virus is added in a proportion of 5 pfu/cell.

When the cytopathogenic effect (CPE) is complete (generally 48 to 72 hours after the beginning of culturing), the viral suspensions are collected then clarified by centrifugation and frozen at -80°C. 3 to 4 successive passages are generally required for the production of a viral batch. The viral batch is stored at -80°C.

### Example 6: Extraction of viral RNA from FIV

The viral RNA contained in 100 ml of viral suspension of the FIV Villefranche strain is extracted after thawing with solutions from the High Pure™ Viral RNA Kit (Cat # 858 882, Roche Molecular Biochemicals), following the supplier's instructions for the extraction stages. The RNA sediment obtained at the end of extraction is resuspended with 1 to 2 ml of sterile distilled water without RNase.

### Example 7: Construction of the plasmid pBP371

The complementary DNA (cDNA) of FIV is synthesised with the Gene Amp RNA PCR Kit (Cat # N 808 0017, Perkin-Elmer, Norwalk, CT 06859, USA), using the conditions provided by the supplier.

A reverse transcription reaction followed by a polymerase chain reaction (RT-PCR) is carried out using 50 µl of the viral RNA suspension of FIV (example 6) and the following oligonucleotides:
FC116 (36 mer) (SEQ ID NO: 6)
   5' TTTTTTCTGCAGCAATAAGAATGGCAGAAGGATTTG 3'
and FC117 (36 mer) (SEQ ID NO: 7)
   5'TCGCACCTGAAACATCTCGAGTGTTTCCACATGTAT 3'

This pair of oligonucleotides allows the incorporation of a Pstl restriction site, a Xhol restriction site and an ATG initiation codon at 5' of the insert.

The synthesis of the first cDNA strand takes place by elongation of the oligonucleotide FC117 after hybridization of the latter with the RNA matrix.

The synthesis conditions of the first cDNA strand are a temperature of 42°C for 15 min, then 99°C for 5 min and finally 4°C for 5 min. The conditions of the PCR reaction in the presence of the pair of oligonucleotides FC116 and FC117 are a temperature of 95°C for 2 min, then 40 cycles (95°C for 30 sec, then 50°C for 45 sec and 72°C for 3 min) and finally 72°C for 7 min to produce a fragment of 1476 bp.

This fragment is digested by the Pstl restriction enzyme then by the Xhol restriction enzyme to isolate the Pstl-Xhol fragment of about 1450 bp after electrophoresis in agarose gel. This fragment is called fragment A.

A second reverse transcription reaction followed by a polymerase chain reaction (RT-PCR reaction) is carried out with 50 µl of the viral RNA suspension of FIV (example 6) and with the following oligonucleotides:
FC118 (36 mer) (SEQ ID NO: 8)
   5' ATACATGTGGAAACACTCGAGATGTTTCAGGTGCGA 3'
and FC119 (54 mer) (SEQ ID NO: 9)
   5' TTTTTTGGATCCCCCGGGCTGCAGGAATTCTGAGATACTTCATCATTCCTCCTC 3'

This pair of oligonucleotides allows the incorporation of a Xhol restriction site, a BamHl restriction site and a stop codon at 3' of the insert.

The synthesis of the first cDNA strand takes place by elongation of the oligonucleotide FC118, after hybridization of the latter with the RNA matrix.

The synthesis conditions of the first cDNA strand are a temperature of 42°C for 15 min, then 99°C for 5 min and finally 4°C for 5 min. The conditions of the PCR reaction in the presence of the pair of oligonucleotides FC118 and FC119 are a temperature of 95°C for 2 min, then 40 cycles (95°C for 130 sec, then 50°C for 45 sec, and 72°C for 3 min) and finally 72°C for 7 min to produce a fragment of 1193 bp.

This fragment is digested by the Xhol restriction enzyme then by the BamHI restriction enzyme to isolate the Xhol-BamHI fragment of about 1170 bp, after electrophoresis in agarose gel. This fragment is called fragment B.

Fragments A and B are ligated with the eukaryotic expression plasmid pVR1012, previously digested by Xbal and EcoRI, to give the plasmid pBP371 (7467 bp). This plasmid contains, under the control of the early promoter of the human cytomegalovirus or hCMV-IE (human Cytomegalovirus Immediate Early), an insert encoding the env protein of FIV.

### Example 8: Construction of the plasmid pBP374

The complementary DNA (cDNA) of FIV is synthesised using the Gene Amp RNA PCR Kit (Cat # N 808 0017, Perkin-Elmer, Norwalk, CT 06859, USA), using the conditions provided by the supplier.

A reverse transcription reaction followed by a polymerase chain reaction (RT-PCR reaction) is carried out with 50 µl of the viral RNA suspension of FIV (example 6) and with the following oligonucleotides:
BP670 (37 mer) (SEQ ID NO: 10)
   5' TTTGTCGACAAGGTAGGAGAGATTCTACAGCAACATG 3'
and BP674 (40 mer) (SEQ ID NO: 11)
   5' TTTGCGGCCGCGTTATTGAGCCATTACTAACCTAATATTG 3'

This pair of oligonucleotides allows the incorporation of a Sall restriction site, a Notl restriction site, an ATG initiation codon at 5' and a stop codon at 3' of the insert.

The synthesis of the first cDNA strand takes place by elongation of the oligonucleotide BP674, after hybridization of the latter with the RNA matrix.

The synthesis conditions of the first cDNA strand are a temperature of 42°C for 15 min, then 9° C for 5 min and finally 4°C for 5 min. The conditions of the PCR reaction in the presence of the pair of oligonucleotides BP670 and BP674 are a temperature of 95°C for 2 min, then 40 cycles (95°C for 30 sec, then 50°C for 45 sec, and 72°C for 3 min) and finally 72°C for 7 min to produce a fragment of 1758 bp.

This fragment is digested by the Sall restriction enzyme then by the Notl restriction enzyme to isolate the Sall-Notl fragment of about 1750 bp, after electrophoresis in agarose gel. This fragment is ligated with the expression plasmid pVR1012, previously digested by Sall and Notl, to give the plasmid pBP374 (6633 bp). This plasmid contains, under the control of the early promoter hCMV-IE, an insert encoding the gag/pro proteins of FIV.

### Example 9: Construction of the plasmid pBP375

The complementary DNA (cDNA) of FIV is synthesised using the Gene Amp RNA PCR Kit (Cat # N 808 0017, Perkin-Elmer, Norwalk, CT 06859, USA), using the conditions provided by the supplier.

A reverse transcription reaction followed by a polymerase chain reaction (RT-PCR reaction) is carried out with 50 µl of the viral RNA suspension of FIV (example 6) and with the following oligonucleotides:
FC116 (36 mer) (SEQ ID NO: 6)
   5'TTTTTTCTGCAGCAATAAGAATGGCAGAAGGATTTG 3'
and FC120 (48 mer) (SEQ ID NO: 12)
   5' TTTTTACCTGCATTTCCTTCTTCCAGTTTTACCTCTTGAATTTCGTTC 3'

This pair of oligonucleotides allows the incorporation of a Pstl restriction site, a BspMI restriction site and an ATG initiation codon at 5' of the insert.

The synthesis of the first cDNA strand takes place by elongation of the oligonucleotide FC120, after hybridization of the latter with the RNA matrix.

The synthesis conditions of the first cDNA strand are a temperature of 42°C for 15 min, then 99°C for 5 min and finally 4°C for 5 min. The conditions of the PCR reaction in the presence of the pair of oligonucleotides FC116 and FC120 are a temperature of 95°C for 2 min, then 40 cycles (95°C for 30 sec, then 50°C for 45 sec, and 72°C for 3 min) and finally 72°C for 7 min to produce a fragment of 265 bp.

This fragment is digested by the restriction enzyme Pstl then by the restriction enzyme BspMI to isolate the Pstl-BspMI fragment of about 240 bp, after electrophoresis in agarose gel. This fragment is called fragment C.

A second reverse transcription reaction followed by a polymerase chain reaction (RT-PCR reaction) is carried out with 50 µl of the viral RNA suspension of FIV (example 6) and with the following oligonucleotides:
BP672 (48 mer) (SEQ ID NO: 13)
   5' TTTACTGGAAGAAGGAAATGCAGGTAAAAGGAAAAGACAAAGAAGAAG 3'
and BP673 (36 mer) (SEQ ID NO: 14)
   5' TTTAGATCTTTAGTCCATAAGCATTCTTTCTATTTC 3'.

This pair of oligonucleotides allows the incorporation of a restriction site BspMI, a restriction site BglII and a stop codon at 3' of the insert.

The synthesis of the first cDNA strand takes place by elongation of the oligonucleotide BP673, after hybridization of the latter with the RNA matrix.

The synthesis conditions of the first cDNA strand are a temperature of 42°C for 15 min, then 99°C for 5 min and finally 4°C for 5 min. The conditions of the PCR reaction in the presence of the pair of oligonucleotides BP672 and BP673 are a temperature of 95°C for 2 min, then 40 cycles (95°C for 30 sec, then 50°C for 45 sec, and 72°C for 3 min) and finally 72°C for 7 min to produce a fragment of 246 bp.

This fragment is digested by the restriction enzyme BspMI then by the restriction enzyme BglII to isolate the BspMI-BglII fragment of about 230 bp, after electrophoresis in agarose gel. This fragment is called fragment D.

Fragments C and D _are_ligated with the expression plasmid pVR1012 previously digested by the restriction enzymes Pstl and BglII to give the plasmid pBP375 (5316 bp). Under the control of the early promoter hCMV-IE, this plasmid contains an insert encoding the rev protein of FIV.

### Example 10: Construction of the plasmid pBP383

The complementary DNA (cDNA) of FIV is synthesised using the Gene Amp RNA PCR Kit (Cat # N 808 0017, Perkin-Elmer, Norwalk, CT 06859, USA), using the conditions provided by the supplier.

A reverse transcription reaction followed by a polymerase chain reaction (RT-PCR reaction) is carried out with 50 µl of the viral RNA suspension of FIV (example 6) and with the following oligonucleotides:
BP680 (29 mer) (SEQ ID NO: 15)
   5' TTTCTGCAGATGGAAGACATAATAGTATT 3'
and BP681 (32 mer) (SEQ ID NO: 16)
   5' TTTAGATCTCTAAGCAGTAGTTATTGATAATG 3'.

This pair of oligonucleotides allows the incorporation of a BglII restriction site, a Pstl restriction site, an initiation ATG codon at 5' and a stop codon at 3' for the insert.

The synthesis of the first cDNA strand takes place by elongation of the oligonucleotide BP681, after hybridization of the latter with the RNA matrix.

The synthesis conditions of the first cDNA strand are a temperature of 42°C for 15 min, then 99°C for 5 min and finally 4°C for 5 min. The conditions of the PCR reaction in the presence of the pair of oligonucleotides BP680 and BP681 are a temperature of 95°C for 2 min, then 40 cycles (95°C for 30 sec, then 50°C for 45 sec, and 72°C for 1 min) and finally 72°C for 7 min to produce a fragment of 254 bp.

This fragment is digested by the Pstl restriction enzyme then by the BglII restriction enzyme to isolate the Pstl-BglII fragment of about 240 bp, after electrophoresis in agarose gel. This fragment is ligated with the expression plasmid pVR1012, previously digested by PstI and BglII, to give the plasmid pBP383 (5089 bp). This plasmid contains, under the control of the early promoter hCMV-IE, an insert encoding the tat protein of FIV.

### Example 11: Extraction of viral RNA from FeLV

Type A FeLV viruses (Glasgow-1 strain) (Steward M. et al., J. Virol., 1986, 58, 825-834) were purified by techniques well known to a person skilled in the art. The genomic viral RNA of each virus was then isolated using the guanidium thiocyanate/phenolchloroform extraction technique described by P. Chomczynski and N. Sacchi (Anal. Biochem., 1987, 162, 156-159).

### Example 12: Construction of the plasmid pBP179

The complementary DNA (cDNA) of FeLV was synthesised using the Gene Amp RNA PCR Kit (Cat # N 808 0017, Perkin-Elmer, Norwalk, CT 06859, USA), using the conditions provided by the supplier.

A reverse transcription reaction followed by a polymerase chain reaction (RT-PCR reaction) is carried out with 50 µl of the viral RNA suspension of FeLV (example 11) and with the following oligonucleotides:
BP247 (29 mer) (SEQ ID NO: 17)
   5' TTTGTCGACCATGGAAAGTCCAACGCACC 3'
and BP249 (28 mer) (SEQ ID NO: 18)
   5' TTTGGATCCTCATGGTCGGTCCGGATCG 3'

This pair of oligonucleotides allows the incorporation of a Sall restriction site, a BamHI restriction site, an ATG initiation codon at 5' and a stop codon at 3' of the insert.

The synthesis of the first cDNA strand takes place by elongation of the oligonucleotide BP249, after hybridization of the latter with the RNA matrix.

The synthesis conditions of the first cDNA strand are a temperature of 42°C for 15 min, then 99°C for 5 min and finally 4°C for 5 min. The conditions of the PCR reaction in the presence of the pair of oligonucleotides BP247 and BP249 are a temperature of 95°C for 2 min, then 40 cycles (95°C for 30 sec, then 50°C for 45 sec, and 72°C for 1 min) and finally 72°C for 7 min to produce a fragment of 1947 bp.

This fragment is digested by the Sall restriction enzyme then by the BamHI restriction enzyme to isolate the Sall-BamHI fragment of about 1935 bp, after electrophoresis in agarose gel. This fragment is ligated with the expression plasmid pVR1012, previously digested by SalI and BamHI, to give the plasmid pBP179 (6804 bp). This plasmid contains, under the control of the early promoter hCMV-IE, an insert encoding the env proteins of FeLV-A.

### Example 13: Construction of the plasmid pBP181

The complementary DNA (cDNA) of FeLV was synthesised using the Gene Amp RNA PCR Kit (Cat # N 808 0017, Perkin-Elmer, Norwalk, CT 06859, USA), using the conditions provided by the supplier.

A reverse transcription reaction followed by a polymerase chain reaction (RT-PCR reaction) was carried out with 50 µl of the viral RNA suspension of FeLV (example 11) and with the following oligonucleotides:
BP283 (33 mer) (SEQ ID NO: 19)
   5' TTGTCGACATGTCTGGAGCCTCTAGTGGGACAG 3'
and BP284 (40 mer) (SEQ ID NO: 20)
   5' TTGGATCCTTATTTAATTACTGCAGTTCCAAGGAACTCTC 3'

This pair of oligonucleotides allows the incorporation of a Sall restriction site, a BamHI restriction site, an ATG initiation codon at 5' and a stop codon at 3' of the insert.

The synthesis of the first cDNA strand takes place by elongation of the oligonucleotide BP284, after hybridization of the latter with the RNA matrix.

The synthesis conditions of the first cDNA strand are a temperature of 42°C for 15 min, then 99°C for 5 min and finally 4°C for 5 min. The conditions of the PCR reaction in the presence of the pair of oligonucleotides BP283 and BP284 are a temperature of 95°C for 2 min, then 40 cycles (95°C for 30 sec, then 50°C for 45 sec, and 72°C for 1 min) and finally 72°C for 7 min to produce a fragment of 3049 bp.

This fragment is digested by the SalI restriction enzyme then by the BamHI restriction enzyme to isolate the Sall-BamHI fragment of about 3039 bp, after electrophoresis in agarose gel. This fragment is ligated with the expression plasmid pVR1012, previously digested by Sall and BamHI, to give the plasmid pBP181 (7908 bp). This plasmid contains, under the control of the early promoter hCMV-IE, an insert encoding the gag/pro proteins of FeLV-A.

### Example 14: Preparation of the plasmids

Any method of obtaining a suspension of purified plasmids may be used to prepare plasmids for the vaccination of cats. These methods are well known to a person skilled in the art. The plasmids are produced by culture of *Escherichia coli* K12 bacteria transformed by plasmids according to the description. These include, in particular, the technique of alkaline lysis followed by two successive ultracentrifugation treatments over a gradient of chloride and caesium in the presence of ethidium bromide as described in Sambrook J. et al., (Molecular Cloning: A Laboratory Manual, 2nd Edition, Cold Spring Harbor Laboratory, Cold Spring Harbor, NY. 1989). Reference may also be made to the patent applications WO-A-95/21250 and WO-A-96/02658 which describe methods of producing plasmids suitable for vaccination on an industrial scale. For the requirements of vaccine production, the plasmids are resuspended so as to provide high-concentration solutions (> 2 mg/ml) compatible with storage. For this purpose, the plasmids are resuspended either in ultrapure water or in a TE buffer (Tris-HCl 10 mM; EDTA 1 mM; pH 8.0).

### Example 15: Production of vaccines and administration

The store of plasmid pBP477 is diluted in TE buffer, in physiological water or in BPS buffer and mixed with various immunogen-expressing vaccinal plasmids, in particular pBP371 (example 7) and/or pBP374 (example 8) and/or pBP375 (example 9) and/or pBP383 (example 10) and/or pBP179 (example 12) and/or pBP181 (example 13). These plasmids may also be, for example, those mentioned in the examples of WO-A-98/03660 and WO-A-00/77043.

The various mixtures of "immunogenic" plasmids and of the plasmid pBP477 thus obtained are co-administered intramuscularly to each cat. In this case, the doses of vaccine are injected in a volume of 1 ml.

### Example 16: Formulation of the plasmids

The mixture of "immunogenic" plasmids and the plasmid pBP477 (example 15) is diluted in TE buffer; physiological water or BPS buffer so as to obtain a concentration of 1 mg/ml. A solution of DMRIE-DOPE at 0.75 mM is prepared by take-up of a lyophilisate of DMRIE-DOPE with a suitable volume of sterile H₂O.

The formation of plamidic DNA-lipid complexes takes place by dilution of the solution of DMRIE-DOPE 0.75 mM in the solution of DNA at 1 mg/ml in equal proportions. The DNA solution is introduced gradually using a crimped needle 26G along the wall of the flask containing the cationic lipid solution so as to prevent foaming. The two solutions are stirred gently after being mixed. A composition containing 0.375 mM of DMRIE-DOPE and 500 µg/ml of DNA is finally obtained.

It is desirable that all the solutions used are at ambient temperature for all the above-described operations. DNA/DMRIE-DOPE complexing is allowed to take place at ambient temperature for 30 minutes before proceeding to immunisation of the animals, as described in example 15.

### Example 17: Construction of the plasmid pCD018

The complementary DNA (cDNA) of FIV is synthesized with the Gene Amp RNA PCR Kit (Cat # N 808 0017, Perkin-Elmer, Norwalk, CT 06859, USA), using the conditions provided by the supplier.

A reverse transcription reaction followed by a polymerase chain reaction (RT-PCR) is carried out using 50 µl of the viral RNA suspension of FIV (example 6) and the following oligonucleotides:
FC118 (36 mer) (SEQ ID NO: 8) and
FC119 (54 mer) (SEQ ID NO: 9).

This pair of oligonucleotides allows the incorporation of a Xhol restriction site, a EcoRI restriction site and a stop codon at 3' of the insert.

The synthesis of the first cDNA strand takes place by elongation of the oligonucleotide FC118, after hybridization of the latter with the RNA matrix.

The synthesis conditions of the first cDNA strand are a temperature of 42°C for 15 min, then 99°C for 5 min and finally 4°C for 5 min. The conditions of the PCR reaction in the presence of the pair of oligonucleotides FC118 and FC119 are a temperature of 95°C for 2 min, then 40 cycles (95°C for 130 sec, then 50°C for 45 sec, and 72°C for 3 min) and finally 72°C for 7 min to produce a fragment of 1193 bp.

This fragment is digested by the Xhol restriction enzyme then by the EcoRI restriction enzyme to isolate the XhoI-EcoRI fragment of about 1150 bp, after electrophoresis in agarose gel. This fragment is called fragment E.

The pCMVβ plasmid (CLONTECH Laboratories Inc., Palo Alto, California, USA, Cat. Number # 6177-1) is digested by the Notl restriction enzyme then by the EcoRV restriction enzyme to isolate the Notl-EcoRV fragment of 3689 bp, after electrophoresis in agarose gel. This fragment is called fragment F.

Fragments A (example 7), E and F are ligated together to give the plasmid pCD018 (6355 bp). This plasmid contains, under the control of the early promoter of the human cytomegalovirus or hCMV-IE (human Cytomegalovirus immediate Early), an insert encoding the env protein of FIV.

### Example 18: Vaccination of cats

Three groups of Hill Grove SPF cats (specific pathogen-free), about 9 to 11 weeks old, were used in the experiment:
- In the control group, 6 cats were injected by intramuscular (IM) route on day 0, day 28 and day 56 with 1.0 ml of physiological solution (NaCl 0.9% in water) containing 0.4 mg of a control plasmid coding for the equine herpesvirus type 1 (EHV-1) glycoprotein C (gC),
- In the second group, 6 cats were injected by intramuscular route on day 0, day 28 and day 56 with 1.0 ml of physiological solution containing 0.1 mg of plasmid pCD018 (example 17), 0.1 mg of plasmid pPB374 (example 8), 0.1 mg of plasmid pPB383 (example 10) and 0.1 mg of a plasmid coding for EHV-1 gC (in order to have the same quantity of injected plasmid),
- In the last group, 6 cats were injected by intramuscular route on day 0, day 28 and day 56 with 1.0 ml of physiological solution containing 0.1 mg of plasmid pCD018 (example 17), 0.1 mg of plasmid pPB374 (example 8), 0.1 mg of plasmid pPB383 (example 10) and 0.1 mg of plasmid pPB477 (example 3).

The three groups were then challenged on day 84 by intramuscular injection in the lumbar muscle of 1.0 ml containing 3.0 log10 CClD50 of FIV strain Petaluma (Pedersen et al., Vet Immunol Immunopathol 1989, 21 (1), 111-29).

After vaccination and after challenge, blood and tissue samples are regularly taken in order to follow the evolution of viral infection, notably to follow the population of CD4 and CD8 lymphocytes, the quantity of FIV proviruses (by PCR) and the quantity of FIV viruses (by RT-PCR).

## Claims

1. An immunogenic composition or vaccine against a feline disease, comprising
1) a feline immunogen or a recombinant vector expressing *in vivo* a feline immunogen, wherein said feline immunogen is a Feline immunodeficiency virus immunogen;
and 2) a polypeptide having an IGF-1 activity, the polypeptide being selected from:
(i) a polypeptide comprising the amino acid sequence as depicted in SEQ ID NO: 2 or SEQ ID NO: 3,
(ii) a polypeptide encoded by nucleotide sequence as depicted in SEQ ID NO: 1,
(iii) a polypeptide comprising amino acids 49-118 of SEQ ID NO: 2,
(iv) a polypeptide having identity equal to or higher than 98.5 % with a polypeptide according to i, ii or iii;
or an *in vivo* expression vector comprising a polynucleotide encoding a polypeptide having an IGF-1 activity, the polynucleotide being selected from:
(i) a polynucleotide comprising a nucleotide sequence as depicted in SEQ ID NO: 1 or its complementary strand,
(ii) a polynucleotide encoding a polypeptide having the amino acid sequence as depicted in SEQ ID NO: 2 or 3,
(iii) a polynucleotide comprising a sequence which differs from the sequence SEQ ID NO: 1 due to the replacement of T by U,
(iv) a polynucleotide encoding amino acids 49-118 of SEQ ID NO: 2,
(v) a polynucleotide encoding a polypeptide having identity equal to or higher than 98.5 % with a polypeptide according to ii or iv;
and 3) a veterinary acceptable vehicle or excipient.

2. The immunogenic composition or vaccine according to claim 1, wherein said immunogenic composition or vaccine further comprises at least one feline immunogen selected from the group consisting of the immunogens of the feline pathogens FeLV, FPV, FIPV, FHV, FCV, rabies virus and *Chlamydia.*

3. The immunogenic composition or vaccine according to claim 1 or 2, wherein the expression vector comprising the polynucleotide is a plasmid.

4. The immunogenic composition or vaccine according to claim 1 or 2, wherein the expression vector comprising the polynucleotide is a viral vector.

5. The immunogenic composition or vaccine according to claim 4, wherein the expression vector comprising the polynucleotide is a poxvirus, an adenovirus or an herpesvirus.

6. The immunogenic composition or vaccine according to any one of claims 1 to 5, wherein the expression vector expressing the immunogen is a plasmid or a viral vector.

7. The immunogenic composition or vaccine according to any one of claims 1 to 5, wherein the expression vector expressing the immunogen is a poxvirus, an adenovirus or an herpesvirus.

8. The immunogenic composition or vaccine according to any one of claims 1 to 5, wherein the immunogen is selected from the group consisting of inactivated immunogen, live attenuated immunogen and sub-units.

9. Use of a composition according to any one of claims 1 to 8 in the preparation of a medicament for the prevention and/or treatment of infection by FIV.

10. Use according to claim 9, wherein said composition is for use in the preparation of a medicament for the prevention and/or treatment of infection by at least one of FeLV, FPV, FIPV, FHV, FCV, rabies virus and *Chlamydia* when said composition further comprises at least one feline immunogen selected from the group consisting of the immunogens of the feline pathogens FeLV, FPV, FIPV, FHV, FCV, rabies virus and *Chlamydia.*

11. A composition according to any one of claims 1 to 8 for use in the prevention and/or treatment of infection by FIV.

12. A composition according to claim 11, wherein said composition is for the prevention and/or treatment of infection by at least one of FeLV, FPV, FIPV, FHV, FCV, rabies virus and *Chlamydia* when said composition further comprises at least one feline immunogen selected from the group consisting of the immunogens of the feline pathogens FeLV, FPV, FIPV, FHV, FCV, rabies virus and *Chlamydia.*

## Patentansprüche

1. Immunogene Zusammensetzung oder Impfstoff gegen eine Katzenkrankheit, umfassend
1) ein felines Immunogen oder einen rekombinanten Vektor, der *in vivo* ein felines Immunogen exprimiert, wobei das feline Immunogen ein Feline Immunschwäche-Virus-Immunogen ist;
und
2) ein Polypeptid, das eine IGF-1-Aktivität aufweist, wobei das Polypeptid ausgewählt ist aus:
(i) einem Polypeptid, das die Aminosäuresequenz wie in SEQ ID NO:2 oder SEQ ID NO:3 dargestellt umfasst,
(ii) einem Polypeptid, das von der Nucleotidsequenz wie dargestellt in SEQ ID NO:1 codiert wird,
(iii) einem Polypeptid, das die Aminosäuren 49-118 der SEQ ID NO:2 umfasst,
(iv) einem Polypeptid, das 98,5% oder mehr Identität mit einem Polypeptid gemäß i, ii oder iii aufweist;
oder einen in vivo-Expressionsvektor, der ein Polynucleotid umfasst, das ein Polypeptid mit einer IGF-1-Aktivität codiert, wobei das Polynucleotid ausgewählt ist aus:
(i) einem Polynucleotid, das eine Nucleotidsequenz wie in SEQ ID NO:1 dargestellt oder ihren komplementären Strang umfasst,
(ii) einem Polynucelotid, das ein Polypeptid codiert, das die Aminosäuresequenz wie in SEQ ID NO:2 oder 3 dargestellt aufweist,
(iii) einem Polynucelotid, das eine Sequenz umfasst, die sich auf Grund der Substitution von T durch U von der Sequenz SEQ ID NO:1 unterscheidet,
(iv) einem Polynucleotid, das die Aminosäuren 49-118 der SEQ ID NO:2 codiert,
(v) einem Polynucleotid, das ein Polypeptid codiert, das 98,5% oder mehr Identität mit einem Polypeptid gemäß ii oder iv aufweist;
und
3) einen veterinärmedizinisch verträglichen Träger oder Exzipienten.

2. Immunogene Zusammensetzung oder Impfstoff nach Anspruch 1, wobei die immunogene Zusammensetzung oder der Impfstoff des Weiteren mindestens ein felines Immunogen umfasst ausgewählt aus der Gruppe bestehend aus den Immunogenen der felinen Pathogene FeLV, FPV, FIPV, FHV, FCV, Tollwutvirus und *Chlamydia.*

3. Immunogene Zusammensetzung oder Impfstoff nach Anspruch 1 oder 2, wobei der das Polynucleotid umfassende Expressionsvektor ein Plasmid ist.

4. Immunogene Zusammensetzung oder Impfstoff nach Anspruch 1 oder 2, wobei der das Polynucleotid umfassende Expressionsvektor ein viraler Vektor ist.

5. Immunogene Zusammensetzung oder Impfstoff nach Anspruch 4, wobei der das Polynucleotid umfassende Expressionsvektor ein Pockenvirus, ein Adenovirus oder ein Herpesvirus ist.

6. Immunogene Zusammensetzung oder Impfstoff nach einem der Ansprüche 1 bis 5, wobei der das Immunogen exprimierende Expressionsvektor ein Plasmid oder ein viraler Vektor ist.

7. Immunogene Zusammensetzung oder Impfstoff nach einem der Ansprüche 1 bis 5, wobei der das Immunogen exprimierende Expressionsvektor ein Pockenvirus, ein Adenovirus oder ein Herpesvirus ist.

8. Immunogene Zusammensetzung oder Impfstoff nach einem der Ansprüche 1 bis 5, wobei das Immunogen ausgewählt ist aus der Gruppe bestehend aus inaktiviertem Immunogen, lebendem attenuiertem Immunogen und Untereinheiten.

9. Verwendung einer Zusammensetzung nach einem der Ansprüche 1 bis 8 in der Herstellung eines Medikaments zur Vorbeugung und/oder Behandlung einer FIV-Infektion.

10. Verwendung nach Anspruch 9, wobei die Zusammensetzung zur Verwendung in der Herstellung eines Medikaments zur Vorbeugung und/oder Behandlung einer Infektion durch mindestens eines aus FeLV, FPV, FIPV, FHV, FCV, Tollwutvirus und *Chlamydia* ist, wenn die Zusammensetzung des Weiteren mindestens ein felines Immunogen ausgewählt aus der Gruppe bestehend aus den Immunogenen der felinen Pathogene FeLV, FPV, FIPV, FHV, FCV, Tollwutvirus und *Chlamydia* umfasst.

11. Zusammensetzung nach einem der Ansprüche 1 bis 8 zur Verwendung in der Vorbeugung und/oder Behandlung einer FIV-Infektion.

12. Zusammensetzung nach Anspruch 11, wobei die Zusammensetzung zur Vorbeugung und/oder Behandlung einer Infektion durch mindestens eines aus FeLV, FPV, FIPV, FHV, FCV, Tollwutvirus und *Chlamydia* ist, wenn die Zusammensetzung des Weiteren mindestens ein felines Immunogen ausgewählt aus der Gruppe bestehend aus den Immunogenen der felinen Pathogene FeLV, FPV, FIPV, FHV, FCV, Tollwutvirus und *Chlamydia* umfasst.

## Revendications

1. Composition immunogène ou vaccin contre une maladie féline, comprenant
1) un immunogène félin ou un vecteur recombinant exprimant un immunogène félin *in vivo,* où ledit immunogène félin est un immunogène du virus de l'immunodéficience féline ; et
2) un polypeptide ayant une activité d'IGF-1, le polypeptide étant choisi parmi :
(i) un polypeptide comprenant la séquence d'acides aminés telle que représentée par SEQ ID NO : 2 ou SEQ ID NO : 3,
(ii) un polypeptide codé par la séquence nucléotidique telle que représentée par SEQ ID NO : 1,
(iii) un polypeptide comprenant les acides aminés 49 à 118 de SEQ ID NO : 2,
(iv) un polypeptide présentant une identité égale ou supérieure à 98,5 % avec un polypeptide selon i, ii ou iii ;
ou un vecteur d'expression *in vivo* comprenant un polynucléotide codant pour un polypeptide ayant une activité d'IGF-1, le polynucléotide étant choisi parmi :
(i) un polynucléotide comprenant une séquence nucléotidique telle que représentée par SEQ ID NO : 1 ou son brin complémentaire,
(ii) un polynucléotide codant pour un polypeptide ayant la séquence d'acides aminés telle que représentée par SEQ ID NO : 2 ou 3,
(iii) un polynucléotide comprenant une séquence qui diffère de la séquence SEQ ID NO : 1 à cause du remplacement de T par U,
(iv) un polynucléotide codant pour les acides aminés 49 à 118 de SEQ ID NO : 2,
(v) un polynucléotide codant pour un polypeptide présentant une identité égale ou supérieure à 98,5 % avec un polypeptide selon ii ou iv ; et
3) un véhicule ou un excipient vétérinaire acceptable.

2. Composition immunogène ou vaccin selon la revendication 1, où ladite composition immunogène ou ledit vaccin comprend en outre un moins un immunogène félin choisi dans le groupe comprenant les immunogènes des agents pathogènes félins FeLV, FPV, FIPV, FHV, FCV, virus de la rage et *Chlamydia.*

3. Composition immunogène ou vaccin selon la revendication 1 ou 2, où le vecteur d'expression comprenant le polynucléotide est un plasmide.

4. Composition immunogène ou vaccin selon la revendication 1 ou 2, où le vecteur d'expression comprenant le polynucléotide est un vecteur viral.

5. Composition immunogène ou vaccin selon la revendication 4, où le vecteur d'expression comprenant le polynucléotide est un virus de la variole, un adénovirus ou un virus de l'herpès.

6. Composition immunogène ou vaccin selon l'une quelconque des revendications 1 à 5, où le vecteur d'expression exprimant l'immunogène est un plasmide ou un vecteur viral.

7. Composition immunogène ou vaccin selon l'une quelconque des revendications 1 à 5, où le vecteur d'expression exprimant l'immunogène est un virus de la variole, un adénovirus ou un virus de l'herpès.

8. Composition immunogène ou vaccin selon l'une quelconque des revendications 1 à 5, où l'immunogène est choisi dans le groupe comprenant un immunogène inactivé, un immunogène vivant atténué et des sous-unités.

9. Utilisation d'une composition selon l'une quelconque des revendications 1 à 8 dans la préparation d'un médicament destiné à la prévention et/ou au traitement d'une infection par le FIV.

10. Utilisation selon la revendication 9, où ladite composition est pour une utilisation dans la préparation d'un médicament destiné à la prévention et/ou au traitement d'une infection par au moins l'un des FeLV, FPV, FIPV, FHV, FCV, virus de la rage et *Chlamydia* lorsque ladite composition comprend en outre au moins un immunogène félin choisi dans le groupe comprenant les immunogènes des agents pathogènes félins FeLV, FPV, FIPV, FHV, FCV, virus de la rage et *Chlamydia.*

11. Composition selon l'une quelconque des revendications 1 à 8, pour une utilisation dans la prévention et/ou le traitement d'une infection par le FIV.

12. Composition selon la revendication 11, où ladite composition est pour la prévention et/ou le traitement d'une infection par au moins l'un des FeLV, FPV, FIPV, FHV, FCV, virus de la rage et *Chlamydia* lorsque ladite composition comprend en outre au moins un immunogène félin choisi dans le groupe comprenant les immunogènes des agents pathogènes félins FeLV, FPV, FIPV, FHV, FCV, virus de la rage et *Chlamydia.*


### SEQUENCE LISTING

<110> MERIAL
<120> feline IGF-1
<130> fIGF-1
<160> 20
<170> PatentIn version 3.0
<210> 1
   <211> 462
   <212> DNA
   <213> Felis catus
<400> 1
   atgggaaaaa tcagcagtct tccaacccaa ttatttaagt gctgcttttg tgatttcttg     60
   aaggtaaaga tgcacatcat gtccccctct catctcttct atctgggcct gtgcttgctc    120
   accttcacca gctctgccac agctggacca gagacgctct gtggggctga gttggtggac    180
   gctcttcagt tcgtgtgtgg agacaggggt ttttatttca acaagcccac ggggtatggc    240
   tccagcagtc ggagggcacc tcagacaggc atcgtggatg agtgctgctt ccggagctgt    300
   gatctgaggc ggctagagat gtactgtgca cccctcaagc ctgccaagtc tgcccgctca    360
   gtccgtgctc agcgccacac tgacatgccc aaggctcaga aggaagtaca tttgaagaac    420
   gcaagtagag ggagtgcggg aaacaagaac tacagaatgt ag                       462
<210> 2
   <211> 153
   <212> PRT
   <213> Felis catus
<400> 2
   Met Gly Lys Ile Ser Ser Leu Pro Thr Gln Leu Phe Lys Cys Cys Phe
   1               5                   10                  15
   Cys Asp Phe Leu Lys Val Lys Met His Ile Met Ser Pro Ser His Leu
               20                  25                  30
   Phe Tyr Leu Gly Leu Cys Leu Leu Thr Phe Thr Ser Ser Ala Thr Ala
           35                  40                  45
   Gly Pro Glu Thr Leu Cys Gly Ala Glu Leu Val Asp Ala Leu Gln Phe
       50                  55                  60
   Val Cys Gly Asp Arg Gly Phe Tyr Phe Asn Lys Pro Thr Gly Tyr Gly
   65                  70                  75                  80
   Ser Ser Ser Arg Arg Ala Pro Gln Thr Gly Ile Val Asp Glu Cys Cys
                   85                  90                  95
   Phe Arg Ser Cys Asp Leu Arg Arg Leu Glu Met Tyr Cys Ala Pro Leu
               100                 105                 110
   Lys Pro Ala Lys Ser Ala Arg Ser Val Arg Ala Gln Arg His Thr Asp
           115                 120                 125
   Met Pro Lys Ala Gln Lys Glu Val His Leu Lys Asn Ala Ser Arg Gly
       130                 135                 140
   Ser Ala Gly Asn Lys Asn Tyr Arg Met
   145                 150
<210> 3
   <211> 105
   <212> PRT
   <213> Felis catus
<400> 3
   Gly Pro Glu Thr Leu Cys Gly Ala Glu Leu Val Asp Ala Leu Gln Phe
   1               5                   10                  15
   Val Cys Gly Asp Arg Gly Phe Tyr Phe Asn Lys Pro Thr Gly Tyr Gly
               20                  25                  30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Lys Pro Ala Lys Ser Ala Arg Ser Val Arg Ala Gln Arg His Thr Asp
   65                  70                  75                  80
   Met Pro Lys Ala Gln Lys Glu Val His Leu Lys Asn Ala Ser Arg Gly
                   85                  90                  95
   Ser Ala Gly Asn Lys Asn Tyr Arg Met
               100                 105
<210> 4
   <211> 39<212> DNA
   <213> Artificial
   <220>
   <223> oligonucleotide
<400> 4
   acgccgtcga catgggaaaa atcagcagtc ttccaaccc 39
<210> 5
   <211> 42
   <212> DNA
   <213> Artificial
   <220>
   <223> oligonucleotide
<400> 5
   cgcggatccc tacattctgt agttcttgtt tcccgcactc cc 42
<210> 6
   <211> 36
   <212> DNA
   <213> Artificial
   <220>
   <223> oligonucleotide
<400> 6
   ttttttctgc agcaataaga atggcagaag gatttg 36
<210> 7
<211> 36
   <212> DNA
   <213> Artificial
<220>
   <223> oligonucleotide
<400> 7
   tcgcacctga aacatctcga gtgtttccac atgtat 36
<210> 8
   <211> 36
   <212> DNA
   <213> Artificial
   <220>
   <223> oligonucleotide
<400> 8
   atacatgtgg aaacactcga gatgtttcag gtgcga 36
<210> 9
   <211> 54
   <212> DNA
   <213> Artificial
   <220>
   <223> oligonucleotide
<400> 9
   ttttttggat cccccgggct gcaggaattc tgagatactt catcattcct cctc 54
<210> 10
   <211> 37
   <212> DNA
   <213> Artificial
   <220>
   <223> oligonucleotide
<400> 10
   tttgtcgaca aggtaggaga gattctacag caacatg 37
<210> 11
   <211> 40
   <212> DNA
   <213> Artificial
   <220>
   <223> oligonucleotide
<400> 11
   tttgcggccg cgttattgag ccattactaa cctaatattg 40
<210> 12
   <211> 48
   <212> DNA
   <213> Artificial
   <220>
   <223> oligonicleotide
<400> 12
   tttttacctg catttccttc ttccagtttt acctcttgaa tttcgttc 48
<210> 13
   <211> 48
   <212> DNA
   <213> Artificial
   <220>
   <223> oligonucleotide
<400> 13
   tttactggaa gaaggaaatg caggtaaaag gaaaagacaa agaagaag 48
<210> 14
   <211> 36
   <212> DNA
   <213> Artificial
   <220>
   <223> oligonucleotide
<400> 14
   tttagatctt tagtccataa gcattctttc tatttc 36
<210> 15
   <211> 29
   <212> DNA
   <213> Artificial
   <220>
   <223> oligonucleotide
<400> 15
   tttctgcaga tggaagacat aatagtatt 29
<210> 16
   <211> 32
   <212> DNA
   <213> Artificial
   <220>
   <223> oligonucleotide
<400> 16
   tttagatctc taagcagtag ttattgataa tg 32
<210> 17
   <211> 29
   <212> DNA
   <213> Artificial
   <220>
   <223> oligonucleotide
<400> 17
   tttgtcgacc atggaaagtc caacgcacc 29
<210> 18
   <211> 28
   <212> DNA
   <213> Artificial
   <220>
   <223> oligonucleotide
<400> 18
   tttggatcct catggtcggt ccggatcg 28
<210> 19
   <211> 33
   <212> DNA
   <213> Artificial
   <220>
   <223> oligonucleotide
<400> 19
   ttgtcgacat gtctggagcc tctagtggga cag 33
<210> 20
   <211> 40
   <212> DNA
   <213> Artificial
   <220>
   <223> oligonucleotide
<400> 20
   ttggatcctt atttaattac tgcagttcca aggaactctc 40